# EUROPEAN PATENT APPLICATION

(11) **EP 2 896 681 A1**
(43) Date of publication of application: **22.07.2015**
(21) Application number: 14151996.7
(22) Date of filing: 21.01.2014
(51) Int. Cl.: C10L 1/02, C07G 1/00, C10G 1/00, C10G 3/00, C12P 7/10, D21C 3/20

(54) **Biorefinery of biomass using irradiation process**

(71) Applicant: Comet AG, 3175 Flamatt (CH)
(72) Inventor: Hommes, Gregor, 79576 Weil am Rhein (DE)
(74) Representative: BOVARD AG

(57) **Abstract**

A biorefinery facility and a method for refining biomass comprises a primary refining process including a mechanical treatment resulting in a size reduction of biomass material, and a secondary refining process including at least one conversion treatment resulting in one or more refined products (A, B, C, D, E, F). The primary refining process comprises a separation treatment, in particular a fractionation treatment, for separating fractions of different components from the biomass material. At least one of the fractions of different components is irradiated for structural modification of said component of the fraction.

## Description

The present invention relates to a method of a biorefinery process and a biorefinery facility for refining biomass including an irradiating process. In particular the present invention relates to the biorefinery of a lignocellulosic feedstock including irradiation by electrons.

Biorefinery is a concept of an industrial process based on the use of Biomass to produce many different kinds of chemical substances and products, like sugars, alcohols, lubricants, polymers, aromatic compounds, and also for producing biofuel, e. g. in the form of bio-ethanol, bio-gas or bio-diesel. The process of biorefinery uses biomass as raw material, which may be based on food and feed grains, forest and agricultural material, marine plants, municipal and industrial waste and side streams, but also on animal material. In the biorefinery process the biomass is mechanically treated in a pre-processing step to reduce and roughly standardize the particle size and to increase the accessible surface area of the material and generate a feedstock for further processing. Subsequently the feedstock is subject to a conversion process, to convert and subsequently separate the desired substances. The conversion process comprises combinations of chemical, physical and biological process steps, including for example catalytic and/or biocatalytic reactions provided by inorganic catalysts and/or biocatalysts, i.e. enzymes, free, aggregated, agglomerated, immobilized or associated to cell fragments and/or whole cells and/or artificial cell systems applied in for instance fermentations.

The pre-processing step may be a mechanical treatment step, like milling, grinding, cutting, pressing and so on. Also the material may be exposed to irradiation, like ion or electron beams. Apart from the size reduction the mechanical treatment may lead to a feedstock that is more susceptible to chemical reactions and/or biocatalysts. Furthermore the pre-processing step may comprise a separation step to separate and divide different components of the raw material. Also the pre-processing step may be repeated in several cycles to prepare the feedstock for further processing.

A widely used raw material for biorefinery is a lignocellulosic biomass material. The lignocellulosic material for example can be provided by cereals, like straw, chaff, stover etc., forestry residues like, reed, reed grass, wood, etc., crops, like corn, sugar cane, and many others. For the biorefinery process the lignocellulosic material may be subject to a fractionation step to prepare the conversion of the predominant components, for example a catalytic or biocatalytic hydrolysis step. The fractionation step basically divides the lignocellulosic material up into the main components cellulose, hemicelluloses and lignin. Thus these main components may be treated in separate further processing steps. For example lignin may pass through an inorganic and/or biological transformation and/or (de)-polymerisation step and cellulose may pass through a enzymatic hydrolyses step.

For example in EP 2008668 A1 a method for producing biofuel is described which focuses on biomass extracted from vegetable polymers. In a pre-processing step the vegetable polymer is smashed and an electron beam is used for decomposing and sterilizing the vegetable polymer. To do so, an electron beam having an energy of 2 MeV to 10 MeV is irradiated to the smashed polymer with a dose of tens to hundreds of kGy during up to ten minutes. With this high energy the chemical structure of the material may be changed and decomposed into monomers. A bond between molecules of cellulose, hemicelluloses and lignin is broken by the electron beam, so that harmful bacteria may be extinct and the vegetable polymer can be decomposed. Afterwards a step for fermenting the decomposed vegetable polymer using enzymes is performed to extract biofuel. The exposure of the vegetable polymers to such high energies by the electron beam can result in a cross-linking effect of the components of the vegetable polymer, in particular of lignin. As a result the effect of enzymes on the vegetable polymer during the fermentation step can be strongly diminished and the amount of applied enzymes has to be increased.

In US 2012/0100577 A1 a further method of refining lignocellulosic biomass materials is disclosed. The biomass material may for example include the entire corn plant, including the corn stalk, corn kernels, leaves and roots. The starting material is mechanically treated, for example by hammermilling, to provide a homogenous, fine material. A thin layer of this feedstock material is treated with a relatively low voltage, high power electron beam radiation. The radiation causes fracturing of bonds in the material, in particular bonding between lignin, cellulose and hemicelluloses is disrupted. It is important that the layer is of uniform thickness and that the material itself is of uniform particle size and density because of the relationship between material thickness and density and penetration depth of the electron beam. Thus the mechanical treatment and preparation of the feedstock has to be fulfilled with great care and it might be necessary to repeat the treatment a plurality of times before the feedstock is ready for conversion. In case of a well prepared thin layer of feedstock of homogenous thickness irradiation can be performed using an electron beam of a nominal energy of less than 1 MeV. Finally the treated feedstock is saccharified into sugars and the sugars are fermented. Again there is the risk of cross-linking effects between the different components of the biomass material before the saccharification and fermentation, which may result in a greater demand for enzymes. Furthermore the pre-processing of the biomass material is lengthy and complex.

It is an object of the present invention to provide a biorefinery method and a biorefinery facility which allow a simple and reliable process flow, reduce the need of additives like enzymes, comprise a low energy consumption, and are suitable for different kind of end products.

These and other objects are fulfilled by a method for refining biomass according to independent claims 1 and a biorefinery facility for refining biomass according to independent claim 13. Advantageous features and preferred embodiments of the biorefinery facility and the method according to the invention are disclosed in dependent claims.

The method for refining biomass according to the invention comprises a primary refining process including a treatment resulting in a size reduction of biomass material and preferably in an optimized accessible surface of the biomass, and a secondary refining process including at least one conversion treatment resulting in one or more refined products. The size reduction treatment preferably is a mechanical treatment like milling, grinding, mashing and the like. But also may be a chemical or thermal treatment like steam explosion or a combination of a mechanical, chemical or thermal treatment. The conversion treatment depends on the requested refined product. For example it may be an enzymatic process to refine sugars, a thermo-chemical process to refine e. g. chemicals, or an acidic process to refine further substances. Also the conversion treatment may comprise a combination of several processes to obtain the desired product.

According to the invention the primary refining process comprises a separation treatment for separating different components from the biomass material, and at least one of these different biomass components is irradiated for structural modification of said component. The single components available by the separation treatment may be pure components, i. e. with only little impurities. But the method according to the invention is also useful for components that may exist as almost pure components, in which not all parts of another component are extracted, but wherein the majority of particles belong to the component to be separated. Such impure components separations may be referred to as fractions. Preferably the irradiation is performed before, advantageously directly before the secondary refining process. Preferably each of the different biomass components is irradiated individually. Thus each of the components can be irradiated using specific energy, dose and/or time parameters and/or additional surrounding parameters (i.e. temperature, humidity, concentration of oxygen, etc.), which are suitable for the respective biomass component and for the requested end product.

A biorefinery facility for refining biomass as described above comprises a primary unit for the primary refining treatment of the biomass and a secondary unit for the secondary refining treatment of the biomass pre-treated in the primary unit. The primary unit comprises a separation unit for extracting different components of the biomass material as mentioned before. At least one of the different components is provided to an irradiation unit for irradiation of said component. The irradiation unit may be in between the primary and the secondary unit or may be part of the primary or the secondary unit.

The selective and discrete irradiation treatment of the separated biomass components allows to realize particular refining requirements for specific end products. Furthermore the energy consumption can be reduced, because the energy can be adapted to the individual needs for treatment of the biomass components instead of irradiating the entire biomass with high intensity irradiation. Also the risk of cross-linking between and within the biomass components is reduced, because the irradiation can be adjusted to avoid cross-linking. Thus compared to conventional biorefinery processes, the method according to the invention requires less additives to counteract cross-linking. Therefore the overall refining process is simplified and less resource consuming.

The irradiation unit may comprise at least one electron beam device, preferably a sealed vacuum tube electron accelerator as electron beam device, which may irradiate at least one of the biomass components by an electron beam. The irradiation unit may comprise several electron beam devices, for example one for each biomass component. Also there may be provided different electron beam devices according to the requirements for irradiation of a specific biomass components. Preferably the electron beam comprises an energy of less than 700 keV, preferably between 80 keV and 600 keV, depending on the type of component. Furthermore the electron beam preferably operates at a power between 1 kW and 1000 kW, more preferred at a range of 2.5 kW - 100 kW, and a low dose of 1 Gy and 100 kGy, more preferred a range of 100 Gy and 25 kGy.

According to one embodiment of the method for refining biomass according to the invention the separation treatment is a fractionation treatment. The fractionation of the biomass components is preferably based on the differentiating physical properties of the different biomass components, for example differing solubility, phase transition properties and the like. The fractionation treatment can separate two or more components from the biomass material. The single components available by the fractionation treatment can be pure components as mentioned above. Mostly a component volume may exist as a fraction of a requested component, but also may comprise negligible amounts of other components or contaminations. For the fractionation treatment for example a fractionation unit is used, which provides a sequence of process steps to separate the individual components.

The method for refining biomass according to the invention is in particular advantageous for refining lignocellulosic biomass. After the size reduction treatment the lignocellulosic biomass is at least separated into a cellulose, a hemicellulose and a lignin component by the separation or fractionation treatment, respectively. As mentioned above these components need not be pure components, but may be a cellulose, a hemicellulose or a lignin rich fraction, in which the predominant component is cellulose, a hemicellulose or a lignin. In particular the fractionation treatment may be an organosolv treatment, which extracts the hemicellulose component and the lignin component according to their specific solubility. The method of the invention is especially suitable for lignocellulosic biomass because lignin has a high reactivity to generate cross-linkings with other components and within itself. Such cross-linkings effects are difficult the crack within the refining process and therefore require special attention. According to the invention each of the components can be treated individually by an electron beam, so that the effect of cross-linking can be adressed by an adequate electron beam. For example a cellulose component is irradiated by an electron beam dose of 0.8 kGy, a hemicelluloses component is irradiated by an electron beam dose of 10 kGy, and/or a lignin component is irradiated by an electron beam dose of 20 kGy.

In the method for refining biomass according to the invention the biomass components may be prepared as a wet film, a spray, a powder or a granulate for irradiation by an electron beam. Preferably all components are irradiated in a wet condition, because drying them is energy intensive. Advantageously the components are prepared in a scavenger solvent like (MeOH, EtOH, etc.) to provide a free radical scavenging activity.

In one embodiment of the method for refining biomass according to the invention the secondary refining process includes a hydrolysis step before a fermentation step, for example for the fermentation of the cellulose component, using a mass ratio of hydrolyzing enzymatic units (U) to the biomass component, e. g. the cellulose component, of 0.1-1000 U per gram dry matter of the component is used. Advantageously a mass ratio of hydrolyzing enzymes to the biomass component below 10 U per gram dry matter is sufficient for good transformation results. Preceding to the hydrolysis step the biomass component is irradiated by an electron beam comprising a dose between 100 Gy and 20 kGy, preferably between 0.8 kGy and 10 kGy, which is particularly advantageous for a cellulose component. That means in the preparation of the fermentation step by the irradiation only a low dose is required, to obtain satisfying results for the final product.

For the method for refining the biomass according to the invention, depending on the type of biomass, it can be advantageous that the primary refining process includes an irradiation step before the separation treatment and/or the secondary refining process includes an irradiation step after a conversion treatment. In this case an irradiation step may be part of the size reduction treatment or the conversion treatment. Such irradiation steps are basically independent of the irradiation of the at least one extracted component or component fraction according to the invention. Although they may be provided by the same irradiation unit of the biorefinery facility. In particular an irradiation step for supporting the mechanical treatment to reduce the size of biomass particles needs much higher irradiation energy and doses as the component irradiation according to the invention. Furthermore the component irradiation may be combined with a catalytic process to support the structural modification of the components. Again the catalytic process is tailored to the requirements of each of the irradiated components.

An exemplary embodiment of the invention will be illustrated in the following drawings, which merely serve for explanation and should not be construed as being restrictive. The features of the invention becoming obvious from the drawings should be considered to be part of the disclosure of the invention both on their own and in any combination. The drawings show:
- Fig. 1:: a basic block diagram for a basic lignocellulosic biorefinery,
- Fig. 2:: a schematical diagram of a method for refining biomass according to the invention, and
- Fig. 3:: a diagram of a conversion efficiency for different process conditions for a cellulose component.

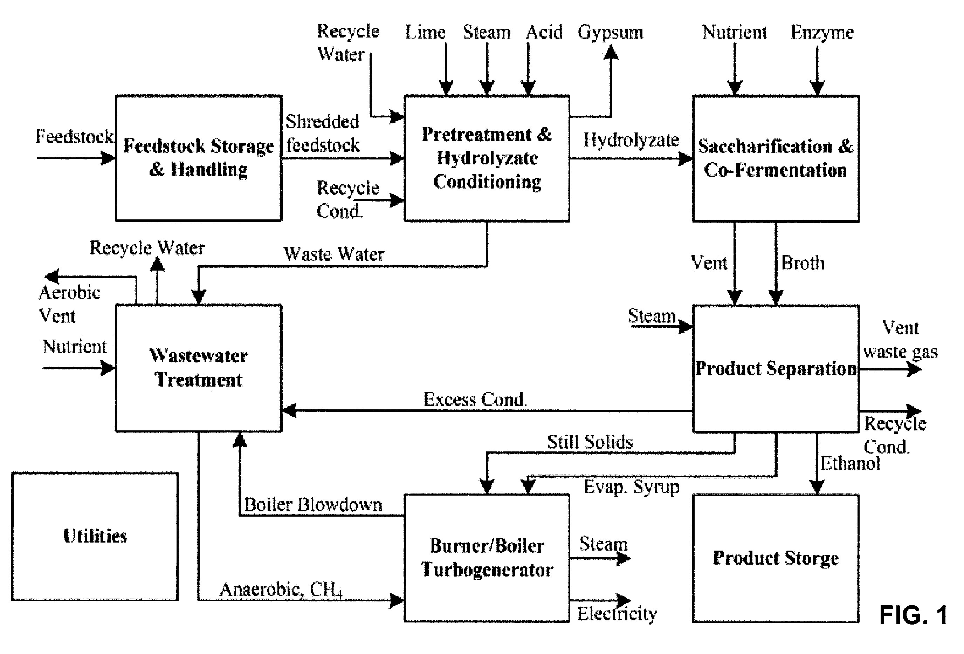

In general biorefinery processes include a large variety of processing steps, wherein the exact process flow is dependent on the substances of the feedstock and refined product, and has to be determined in accordance to the particularities of the designated substance and product. As exemplification of a biorefinery processes that e. g. might be used as a basis for a method for refining biomass according to the invention, figure 1 shows a basic block diagram for a basic lignocellulosic biorefinery as disclosed in Appl. Biochem. Biotechnol, 2009, Vol 154: 205 - 216. Any information on the individual steps of lignocellulosic biorefinery as described therein can be used to describe the basic steps, which can be used in combination with the method for refining biomass according to the invention.

In summary conventional biorefinery processes involving conversion of lignocellulosic feedstock into bioethanol comprises the following steps: feedstock handling, pre-treatment and conditioning and detoxification, saccharification and co-fermentation, product separation and purification, wastewater treatment, product storage, lignin combustion e g. for production of electricity and steam. More information on the biorefinery processes is given in figure 1, for example in respect of processing sequences, supplements used for the processing steps and intermediate and end products of the processing steps.

In the following the method for refining biomass according to the present invention will be described for a lignocellulosic biomass, which is separated into three component fractions: cellulose fraction, hemicellulose fraction and lignin fraction. As mentioned before a component fraction consists of a requested predominant component, but may comprise negligible amounts of other components, that are present in the biomass material. It is clear to a person skilled in the art, that the method can advantageously be applied to other biomass materials, which will be separated into different types of component fractions. Certainly, also only two or more than three biomass component fractions may be extracted from a biomass material, for example lipids, proteins or waxes. Therefore the lignocellulosic biomass is only one of many examples of a biomass material that may be refined into different kind of products.

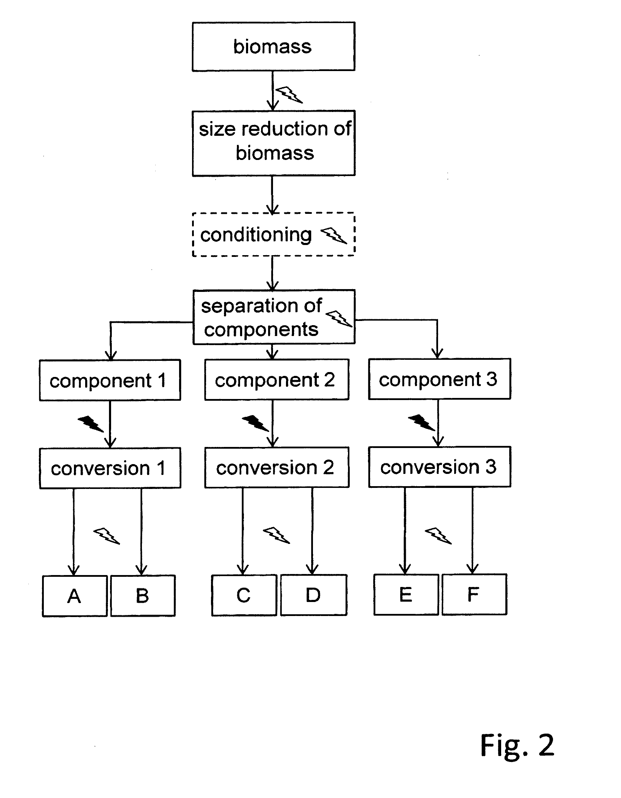

Figure 2 shows a flow chart for the method for refining the lignocellulosic biomass according to the present. The lignocellulosic biomass may consist of forest, agricultural or marine plant material, parts thereof, waste material, like paper, cardboard, tissues, and mixtures thereof. Basically any kind of lignocellulosic biomass may be used.

In general within the description of the method for refining biomass according to the figure 2 irradiation steps shown as white arrows shall be understood as optional irradiation. The irradiation treatment depicted as black arrows refer to an advantageous irradiation of different biomass component fractions according to the invention.

The lignocellulosic biomass is fed into the primary refining process, where it is subject to a mechanical treatment to reduce the size of the biomass material that means to reduce the size of the particles of the biomass material. The mechanical treatment may, for example, consist of a step of milling, grinding, chopping, shearing, cutting, pressing as commonly know. The mechanical treatment in general is chosen according to the type of biomass material. After the size reduction process the biomass material has a size between 1 nm and 10 mm, preferably between 0.001 and 2 mm. In the following the size reduced material will be referred to as a feedstock for the further processing in the biorefinery method.

Before, during or after the size reduction treatment the lignocellulosic biomass or the feedstock optionally may be treated by an irradiation step, which may decrease the number of active microorganisms disturbing the refining process, stabilize the biomass and prevent undesired transformation like spontaneous fermentation of soluble sugars. The irradiation step is for example performed by an electron beam comprising an energy of 80 kV - 600 kV.

Also optionally a conditioning step with or without an irradiation step may be applied after the size reduction treatment to the feedstock. The conditioning step can prepare the feedstock for a following conversion or separation treatment as commonly known. Applying an irradiation step to the feedstock for conditioning may for example increase the wettability or facilitate the migration of compounds in the pretreated material.

The primary refining process also includes a separation treatment for extracting different components fractions of the feedstock. In the example of the lignocellulosic biomass feedstock the separation treatment is a fractionation treatment, which is achieved by an organosolv process. The organosolv process is particularly useful for extracting the lignin component from the feedstock, and also allows to extract the cellulose component fraction and the hemicellulose component fraction. An organosolv treatment for lignocellulosic biomass is commonly known and for example described in Sun, Y., Cheng, J., 2002, Hydrolysis of lignocellulosic materials for ethanol production: a review; Bioresource Technology, 83:1-11.

In general in the organosolv process, an organic or aqueous organic solvent mixture with inorganic acid catalysts, likehydrochloric acid or sulfuric acid, is used to break the internal lignin and hemicellulose bonds. The organic solvents used in the process include methanol, ethanol, acetone, ethylene, glycol, triehylene glycol, and tetrahydrofurfuryl alcohol. Typical process conditions are for example between 160 °C and 200 °C for 15 min to 120 min at a pressure between 5 bar and 30 bar. The solvent may be separated from the extracted components and recycled to the process. As shown in figure 2 the feedstock is separated into a fraction of component 1, a fraction of component 2 and a fraction of component 3, which correspond to the cellulose component fraction, the hemicellulose component fraction and the lignin component fraction. Advantageously an irradiation step for irradiating the feedstock can assist the processes during the fractionation treatment. For example the irradiation step increases the solubility of the conditioned feedstock and facilitates the migration and precipitation of the components of the feedstock. Furthermore the irradiation can provide for sterilization of the components. The irradiation step during the fractionation treatment is for example performed by an electron beam comprising an energy of 25 kGy.

The different extracted components 1, 2, and 3 are prepared for a subsequent irradiation treatment for a structural modification of the components. For example the components are prepared as a thin wet film comprising a thickness between 0.1 mm - 5 mm or as a spray with droplets comprising a size between 0.01 mm - 5 mm. Alternatively the components are prepared as a powder with a particle size between 10 nm and 0.1 mm or granules with a particle size between 0.1 mm and 10 mm in a dry condition. Preferably the cellulose component fraction is prepared in a wet or dry condition in form of 0.5-1 mm granules, the hemicellulose component fraction is prepared in a wet or dry condition in form of 0.5-1 mm granules, and the lignin component fraction is prepared in a wet or dry condition in form of powder or particle suspension provided to the beam as a thin film.

Before a secondary refining process for refining the single biomass components to different sorts of end products, the prepared component fractions are individually submitted to an irradiation treatment for structural modification of said components. The structural modification changes the characteristics of the components and therefore supports the conversion treatment of the secondary refining process. The irradiation treatment takes into account the particularities of the single biomass components for the configuration of the irradiation. In general lower energies and doses can be used compared to the irradiation steps as mentioned before, because the configuration can focus on a specific component and a specific conversion treatment for said component. Mostly doses between 1 Gy and 10 kGy or between 80 keV and 600 keV is sufficient. Advantageously for the transition of cellulose a lower dose may be used than for the transition of lignin. Preferably the irradiation treatment is carried out in combination with a subsequently applied catalytic step. For each of the components an appropriate catalytic medium is selected, for example oxidoreductases, like laccases or peroxidases, singular or in combination of each other can be applied in solvents like dimethylsolfoxide (DMSO), alcohols, ionic liquids, water, etc. or solvent mixtures like DMSO/water in appropriate ratios.

The electron beam may be provided by an irradiation unit comprising one or more electron beam sources with a treatment window width between 200 mm and 1000 mm.

In the secondary refining process each of the component fractions is subjected to a conversion treatment, which refines one or more end products A, B, C, D, E, and F from the single biomass components. The conversion treatment may be a fermentation, a synthesis, a (de)polymerisation and the like. For example the cellulose component fraction is subject to an enzymatic hydrolysis for fermentation into end products like glucose, ethanol, butanol, propanediol, lactic acid, or acetone, or for conversion into lubricants and other chemicals and polymers. The lignin component fraction may be subject to a thermo-chemical (de)polymerisation conversion into natural binders and adhesives, phenolics, phenolics, styrene, carbon fibers or electricity and heat in form of bituminous coal or sulphur-free solid fuel. The hemicellulose component fraction may be subject to a hydrolysis for conversion into xylose, xylite, furfural, etc.

In general the refining conversion treatment may involve further steps like concentration, distillation, and the like as known in the art of biorefinery. After the conversion treatment a further irradiation step may be applied to the products for example for sterilization or stabilization to prevent undesired transformations.

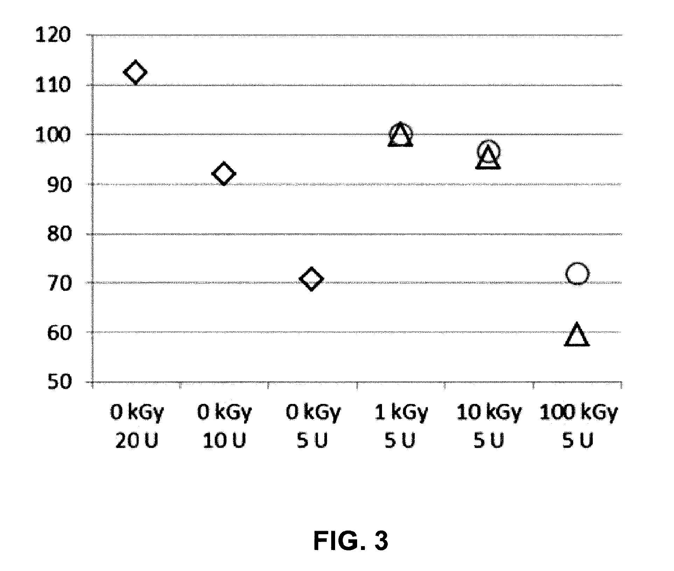

In figure 3 a diagram is shown, which describes experimental results gained for a refining process of a cellulose component fraction subjected to an irradiation for structural modification of the cellulose component after the fractionation of the cellulose according to the invention, and for a refining process of a cellulose component fraction without such irradiation according to the state of the art. In the diagram the vertical axis shows a percentage of component conversion and the horizontal axis indicates the dose in Kilogray [kGy] and the units [U] of enzymes per [gram, dry matter] of cellulose component fraction used for fermentation of the cellulose. The squares indicate the results for an amount of 20, 10 and 5 units of enzymes and no irradiation after fractionation, i. e. zero kGy. As can be seen a high amount of enzymes results in a high conversion percentage: 110% for 20 units of enzymes and 90% for 10 units of enzymes. But a low amount of enzymes of only 5 units results in only 70% of converted cellulose component.

The triangles indicate a dry cellulose component fraction and the circles indicate a wet cellulose component fraction, each sample provided with only 5 units of enzymes and subjected to different electron beam irradiation. As can be seen low doses of 1 kGy and 10 kGy result in a conversion percentage of 95% to 100%, which exceeds the result of the sample with 10 units enzymes and no irradiation. That means the irradiation after the fractionation reduces the amount of enzymes required for the conversion treatment of the cellulose component. Samples of cellulose component fractions irradiated with a dose of 100 kGy shows a fewer conversion percentage of 60% and 70%, respectively. That means that the conversion is improved by using low dosage of electron beams, while high dosages are counterproductive.

Summarized these results proof that a method for refining a lignocellulosic biomass according to the invention can reduce the need of enzymes during hydrolysis before fermentation, comprises a low energy consumption, and simplifies the process flow.

## Claims

1. Method for refining biomass comprising a primary refining process including a treatment resulting in a size reduction of biomass material, and a secondary refining process including at least one conversion treatment resulting in one or more refined products,
wherein the primary refining process comprises a separation treatment for extracting fractions of different components from the biomass material, and
wherein at least one of the fractions of different components is irradiated for structural modification of said component of the fraction.

2. Method for refining biomass according to claim 1, wherein each of the different biomass component fractions is irradiated individually.

3. Method for refining biomass according to claim 1 or 2, wherein at least one of the biomass component fractions is irradiated by an electron beam, in particular a sealed vacuum tube electron accelerator.

4. Method for refining biomass according to the preceding claim, wherein the electron beam comprises an energy of less than 700 keV, preferably between 80 keV and 600 keV.

5. Method for refining biomass according to one of the preceding claims, wherein the electron beam operates at a power between 1 kW and 1000 kW, preferably between 2 kW and 100 kW.

6. Method for refining biomass according to one of the preceding claims, wherein the separation treatment is a fractionation treatment, in particular an organosolv treatment.

7. Method for refining biomass according to one of the preceding claims, wherein a lignocellulosic biomass is used, which is at least separated into a cellulose, a hemicellulose and a lignin component fraction.

8. Method for refining biomass according to the preceding claim, wherein a cellulose component is irradiated by an electron beam of a lower dose than a lignin component.

9. Method for refining biomass according to one of the preceding claims, wherein a biomass component is prepared as a wet film, a spray, a powder or a granulate and irradiated by an electron beam.

10. Method for refining biomass according to one of the preceding claims, wherein the secondary refining process includes a hydrolysis step before a fermentation step using a mass ratio of hydrolyzing enzymatic units (U) to biomass component of 0.1-1000 U per gram dry matter of the component.

11. Method for refining biomass according to the preceding claim, wherein the biomass component is irradiated by an electron beam comprising a dose between 100 Gy and 20 kGy, preferably between 0.8 kGy and 10 kGy.

12. Method for refining biomass according to one of the preceding claims, wherein the primary refining process includes an irradiation step before the separation treatment and/or the secondary refining process includes an irradiation step after a conversion treatment.

13. Biorefinery facility for refining biomass according to a method of one of the preceding claims, comprising a primary unit for a primary refining treatment of a biomass and a secondary unit for a secondary refining treatment of the pre-treated biomass, wherein the primary unit comprises a separation unit for separating fractions of different components of biomass material, wherein at least one of the different component fractions is provided to an irradiation unit for irradiation of said component of the fraction.

14. Biorefinery facility according to claim 13, wherein the irradiation unit comprises at least one vacuum electron beam device, in particular a sealed vacuum tube electron accelerator.
